**Europäisches Patentamt**

**European Patent Office**

**Office européen des brevets**

(19)

(11) Veröffentlichungsnummer: **0 025 044**
**B1**

(12) **EUROPÄISCHE PATENTSCHRIFT**

(45) Veröffentlichungstag der Patentschrift:
02.05.85

(51) Int. Cl.⁴: **A 01 N 59/26, A 01 N 25/18**

(21) Anmeldenummer: **80900465.8**

(22) Anmeldetag: **10.03.80**

(86) Internationale Anmeldenummer:
**PCT/DE 80/00026**

(87) Internationale Veröffentlichungsnummer:
**WO 80/01866 (18.09.80 Gazette 80/21)**

(54) **BEUTEL FÜR SCHÄDLINGSBEKÄMPFUNGSMITTEL.**

(30) Priorität: **10.03.79 DE 7906765 U**

(43) Veröffentlichungstag der Anmeldung:
**18.03.81 Patentblatt 81/11**

(45) Bekanntmachung des Hinweises auf die Patenterteilung:
**02.05.85 Patentblatt 85/18**

(84) Benannte Vertragsstaaten:
**FR**

(56) Entgegenhaltungen:
**DE - A - 2 414 548**
**DE - B - 2 206 494**
**DE - C - 698 721**
**FR - A - 2 262 631**
**FR - A - 2 290 843**
**US - A - 3 719 751**

**L. KREFT IN "KUNSTSTOFFE" Bd. 15, 1955, Seite 274**

**Die Akte enthält technische Angaben, die nach dem Eingang der Anmeldung eingereicht wurden und die nicht in dieser Patentschrift enthalten sind.**

(73) Patentinhaber: **DR. WERNER FREYBERG, CHEMISCHE FABRIK, DELITIA NACHF., Bergstrasse, D-6941 Laudenbach (DE)**
Patentinhaber: **HAUFE, Ludwig, Schollstrasse 17, D-6940 Weinheim (DE)**

(72) Erfinder: **HAUFE, Ludwig, Schollstrasse 17, D-6940 Weinheim (DE)**
Erfinder: **FRIEMEL, Wolfgang, Giessenerstrasse 4, D-6148 Heppenheim (DE)**
Erfinder: **EHRET, Reiner, Ahornstrasse 78, D-6940 Weinheim (DE)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

## Beschreibung

Die Erfindung betrifft einen Beutel zur Aufnahme Phosphinentwickelnder Schädlingsbekämpfungsmittel aus einem gasdurchlässigen wasserfreien Gewebe aus synthetischen Fasern.

Es sind Schädlingsbekämpfungsmittel fester Konsistenz bekannt, die an der Luft langsam gasförmige Bestandteile abgeben. Solche Schädlingsbekämpfungsmittel können aus hydrolisierbaren Erdalkali- und/oder Erdmetallphosphiden bestehen, die unter dem Einfluss von Luft bzw. Lagergutfeuchtigkeit Phosphin abspalten. Derartige Schädlingsbekämpfungsmittel werden beispielsweise zur Schädlingsbekämpfung in Lagerhäusern bzw. Silos für Getreide, Tabak oder ähnlichen Nahrungs-bzw. Genussmitteln eingesetzt. Das Schädlingsbekämpfungsmittel dient dabei der Vernichtung von tierischen Organismen, wie z.B. Käfern, Würmern, Schaben oder sonstigen Insekten. Es ist bekannt, hierfür derartige Schädlingsbekämpfungsmittel in Papierbeutel zu füllen und in das Lagergut einzubringen. Das Schädlingsbekämpfungsmittel sollte dann in dem Papierbeutel langsam zerfallen und seine schädlingsbekämpfenden, gasförmigen Bestandteile durch die Wände des Papierbeutels nach aussen entlassen. Die staubförmigen Rückstände des Schädlingsbekämpfungsmittels sollen dabei von dem Papierbeutel zurückbehalten werden. An das hierfür verwendete Papier werden besondere Anforderungen bezüglich Festigkeit, Gasdurchlässigkeit und wasserabweisender Ausrüstung gestellt.

Solche Papiere können aber nur sehr bedingt auf automatischen Anlagen (wie z.B. Schlauchbeutelmaschinen etc.) verarbeitet werden. Sie werden zum überwiegenden Teil durch Nähen verschlossen, was einen hohen Arbeitsaufwand erfordert. Auch kann es leicht vorkommen, dass die Nähte aufgehen und der giftige Inhalt in das zu behandelnde Nahrungsmittel gelangt.

In der deutschen Patentschrift 2 414 548 ist weiterhin beschrieben, dass Metallphosphid-enthaltende Schädlingsbekämpfungsmittel in einem Beutel mit einem Verschlussmaterial aus bestimmten natürlichen oder synthetischen Polymeren verpackt sein können. An einer Stelle der Beschreibung ist zwar angegeben, dass das Beutelmaterial ein Faservlies sein kann; jedoch ist diese Lehre widersprüchlich, da das Faservlies ein spezielles Beispiel für ein reissfestes Gewebe darstellen soll, während diese Begriffe tatsächlich Materialien bezeichnen, die sich gegenseitig ausschliessen. Die in dieser Patentschrift beschriebenen Beutel zeigen ferner auch hinsichtlich der Verschweissbarkeit des Beutelmaterials einen speziellen, anderen Anforderungen genügenden, aber aufwendigen Lösungsweg. Im Zusammenhang mit der Möglichkeit, die bekannten Beutel beispielsweise durch Wärmeeinwirkung zu verschweissen, ist ausdrücklich die Verwendung eines zusätzlichen Verschlussmaterials vorgesehen. Eine Wärmeverschweissung ohne die Verwendung eines derartigen Verschlussmaterials, also beispielsweise die Herstellung eines Beutels durch direkte Verschweissung zweier Vliesstoff-

Folien durch Wärme und/oder Ultraschall zur Herstellung des Beutels wird in dieser Patentschrift nicht beschrieben. In jedem beschriebenen Fall sind auch das Material, aus dem der Beutel hergestellt worden ist, und das Verschlussmaterial grundsätzlich verschieden. Schliesslich sind in dieser Patentschrift keinerlei Bedingungen hinsichtlich bestimmter Titerwerte sowie Grenzen der Maschenweite des Vliesstoffes gemacht worden.

Der Erfindung liegt die Aufgabe zugrunde, einen Beutel aus einem Material zu schaffen, der eine für die Anwendung erforderliche Gasdurchlässigkeit aufweist, jedoch die staubförmigen Rückstände des zerfallenen Schädlingsbekämpfungsmittels zurückbehält und zusätzlich noch eine hohe Haltbarkeit besitzt, wobei auch eine grosse Sicherheit gegenüber dem Austritt von Feststoffteilchen des Schädlingsbekämpfungsmittels aufgrund von Undichtigkeit oder Beschädigung der Schweissnähte vor oder während dessen Verwendung gegeben ist. Ferner sollen die erfindungsgemässen Beutel auf handelsüblichen automatischen Anlagen erfolgreich und ökonomisch hergestellt, gefüllt und/oder verschlossen werden können.

Gegenstand der Erfindung ist ein Beutel zur Aufnahme Phosphin-entwickelnder Schädlingsbekämpfungsmittel aus einem gasdurchlässigen wasserfreien Gewebe aus synthetischen Fasern, der dadurch gekennzeichnet ist, dass der Beutel durch Verschweissen eines wasserfreien Vlieses mittels Wärme und/oder Ultraschall hergestellt ist, wobei das Vlies eine Maschenweite von 5–15 μ, vorzugsweise 10–12 μ, aufweist, und die Fasern des Vliesstoffes einen Titer von 1–3, vorzugsweise 1,5–2, zeigen.

Der Austritt der Rückstände der Schädlingsbekämpfungsmittel wird von einem solchen Vliesstoff praktisch vollkommen verhindert.

Zur Herstellung der erfindungsgemässen Beutel kann Vliesstoff von einer Vliesstoffbahn entsprechend gefaltet und an den Längsseiten verschweisst werden. In die verbleibende Öffnung lässt sich nun das feste, gewöhnlich granulierte Schädlingsbekämpfungsmittel einfüllen. Danach wird auch noch die verbliebene Öffnung gegebenenfalls durch Schweissen verschlossen.

Es ist möglich, den Vliesstoff auf einer automatischen Schlauchbeutelmaschine in an sich bekannter Weise zu fertigen und gleichzeitig zu befüllen.

Als Material für den erfindungsgemässen Beutel eignen sich Vliesstoffe, und zwar Trockenvliese oder Nassvliese. Sie sind aus verschweissbaren synthetischen Fasern, insbesondere handelsüblichen Fasern aus Polyester, Polyäthylen, PVC, Polypropylen und Polyamid. Diese sollen weder oberflächlich feucht sein, noch während des Gebrauchs aus dem Material abspaltbare Feuchtigkeit aufweisen, ggf. müssen die Materialien getrocknet werden.

Bei einer bevorzugten Ausführungsform besteht das Material für den erfindungsgemässen Beutel aus Wirrvliesstoff. Zweckmässige Abmessungen des erfindungsgemässen Beutels richten sich selbstverständlich nach der gewünschten Menge des aufzunehmenden Schädlingsbekämpfungsmittels.

Bei einer bevorzugten Ausführungsform besteht der Vliesstoff aus Polyamidfasern. Solche Vliesstoffbahnen aus entsprechendem Material werden bei der Faltung zu Beuteln bzw. Formung zu Schlauchbeuteln an den Seiten nicht verklebt, sondern durch Wärme und/oder Ultraschall in an sich bekannter Weise verschweisst. Für besondere Einsatzzwecke können auch mehrere Beutel zu einer Beutelkette verbunden werden.

Selbstverständlich kann auch ein Mehrfachbeutel bzw. ein Beutel mit mehreren Kammern hergestellt werden. Dabei dienen Zwischennähte zur Unterteilung in Kammern bzw. Segmente. Doppelbeutel können beispielsweise einen gemeinsame Quernaht am Ende der Längsseiten 2 besitzen.

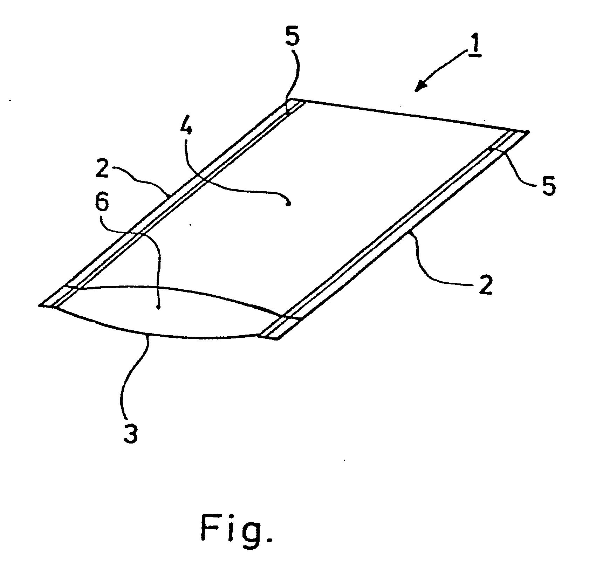

Ein Ausführungsbeispiel der Erfindung ist in der beiliegenden Zeichnung dargestellt.

Der Beutel 1 weist eine Unterseite 3 und eine Oberseite 4 auf, wobei erstere am offenen Ende des Beutels etwas hervorsteht. An den Längsseiten 2 befindet sich eine Verbindungsnaht 5. Es bleibt eine Öffnung 6, durch die das Schädlingsbekämpfungsmittel eingeführt werden kann.

Wie bereits kurz angegeben, besteht eine zweckmässige Herstellungsweise eines solchen Beutels darin, dass die Vliesstoffbahn derart gefaltet wird, dass die Oberseite 4 auf die Unterseite in der angegebenen Weise zu liegen kommt, wonach man die Längsseiten 2 verschweisst. Nach der Füllung wird der hervorstehende Abschnitt der Unterseite 3 gefaltet und auf die Oberseite 4 gelegt, wonach auch diese Querseite in der angegebenen Weise verschweisst werden kann. Nach dem Einschluss in den erfindungsgemässen Beutel ist das Schädlingsbekämpfungsmittel vollkommen eingeschlossen und die bei dem Zerfall von diesem gebildeten Stäube können aus dem Beutel nicht mehr austreten. Somit kann der Beutel mit dem Schädlingsbekämpfungsmittel direkt zu den zu begasenden Lebensmitteln gegeben werden. Der Vorzug des erfindungsgemässen Schädlingsbekämpfungsmittelbeutels liegt darin, dass er praktisch völlig gadurchlässig ist, dennoch aber die staubförmigen Rückstände der zerfallenen Schädlingsbekämpfungsmittels zurückhält. Ein erfindungsgemässer Beutel für Schädlingsbekämpfungsmittel besitzt darüberhinaus eine verbesserte Haltbarkeit und einen äusserst geringen Feuchtigkeitsgehalt. Dadurch wird eine vorzeitige unerwünschte Gasentwicklung bei der Herstellung verhindert.

## Patentansprüche

1. Beutel zur Aufnahme Phosphin-entwickelnder Schädlingsbekämpfungsmittel aus einem gasdurchlässigen wasserfreien Gewebe aus synthetischen Fasern, dadurch gekennzeichnet, dass der Beutel durch Verschweissung eines wasserfreien Vlieses mittels Wärme und/oder Ultraschall hergestellt ist, wobei das Vlies eine Maschenweite von 5–15 μ, vorzugsweise 10–12 μ, aufweist, und die Fasern des Vliesstoffes einen Titer von 1–3, vorzugsweise 1,5–2, zeigen.

2. Beutel nach Anspruch 1, dadurch gekennzeichnet, dass es sich bei dem Vlies um einen Wirrvliesstoff handelt.

3. Beutel nach Anspruch 1–2, dadurch gekennzeichnet, dass die Seiten einer zusammengefalteten Vliesstoffbahn verschweisst sind.

4. Beutel nach Anspruch 1–2, dadurch gekennzeichnet, dass er als Schlauchbeutel ausgebildet ist.

5. Beutel nach Anspruch 1–4, dadurch gekennzeichnet, dass er mit einer Phosphin-entwickelnden Zubereitung von Erdalkali- und/oder Erdmetallphosphiden gefüllt ist.

## Claims

1. Bag to contain phosphine-developing pesticides made of a gas permeable anhydrous material of synthetic fibers, characterized in that the bag has been produced by sealing an anhydrous fleece by means of heat and/or ultrasonics, the fleece having a mesh aperture of 5 to 15 μ, preferably 10 to 12 μ, and the fibers of the fleece material showing a titer o of 1 to 3, preferably 1.5 to 2.

2. Bag according to claim 1, characterized that the fleece is a random fleece material.

3. Bag according to claims 1 and 2, characterized in that the sides of a foldet length of the fleece material are sealed.

4. Bag according to claims 1 and 2, characterized that it is made as a tubular bag.

5. Bag according to claims 1 to 4, characterized in that it is filled with a phosphine-developing composition of alkali earth and/or earth metal phosphides.

## Revendications

1. Sac de conditionnement pour un produit antiparasites qui dégage de la phosphine, constitué d'un tissu perméable aux gaz et exempt d'humidité, à base de fibres synthétiques, caractérisé en ce qu'il est confectionné par soudage à chaud et/ou par ultra-sons à partir d'un non tissé exempt d'humidité, ce non tissé comportant des mailles de 5 à 15 microns, de préférence des mailles de 10 à 12 microns; et en ce que les fibres de tissu non tissé ont un titre de l à 3, de préférence de 1,5 à 2.

2. Sac selon la revendication 1, caractérisé en ce que le tissu non tissé présente und texture emmêlée.

3. Sac selon l'une des revendications 1 ou 2, caractérisé en ce que les côtés d'une bande de tissu non tissé repliée sont assemblés par soudage.

4. Sac selon l'une des revendications 1 ou 2, caractérisé en ce qu'il est conformé en forme de sachet.

5. Sac selon l'une des revendications 1 à 4, caractérisé en ce qu'il est rempli d'une préparation de phosphures alcalino-terreux et/ou de phosphures de métaux terreux, destinée à dégager de la phosphine.

Fig.